# EUROPEAN PATENT SPECIFICATION

(11) **EP 0 428 567 B1**
(45) Date of publication and mention of the grant of the patent: **15.05.1996**
(21) Application number: 89908773.8
(22) Date of filing: 28.07.1989
(51) Int. Cl.: A61B 17/02

(54) **IMPROVED SPINAL RETRACTOR**
RÜCKGRATRETRAKTOR
RETRACTEUR SPINAL AMELIORE

(30) Priority: 29.07.1988 US 225922
(43) Date of publication of application: 29.05.1991
(73) Proprietor: MICHELSON, Gary Karlin, Venice, CA 90291 (US)
(72) Inventor: MICHELSON, Gary Karlin, Venice, CA 90291 (US)
(74) Representative: Schmitz, Jean-Marie
(86) International application number: US8903215
(87) International publication number: WO9001298

(56) References cited:
- DE-U- 8 802 118
- DE-U- 8 813 652
- US-A- 2 053 868
- US-A- 2 450 194
- US-A- 2 642 862
- US-A- 3 766 910
- US-A- 4 627 421
- US-A- 4 747 394

## Description

The present invention relates to a surgical retractor.

### BACKGROUND

The performance of surgery requires that a retractor hold out of the way all tissues down to the actual surgical site. When performing a posterior approach to the thoracic or lumbar spine, it is necessary to retract very large and strong paraspinal musculature masses. Because of the differences in the size of patients and the relative mass of the muscles along various portions of the spine, it would be necessary to have a series of retractors with permanently attached blades or a series of blades that could be attached or removed as needed from a basic blade-holding retractor frame. However, to keep the retractor blades from ejecting from the wound under the pressures generated by the muscle retraction, it is necessary to have the blades both diverge from the center line distally, and further to have large sharp teeth at a right angle to the longitudinal access of the blades so as to lock the blade tips beneath the muscle masses.

If the blades are divergent distally and in addition are splayed, then even with the retractor fully closed, the blades, if pre-attached to the retractor, would not be insertable into the wound as the outside of the tips of the blades would be further apart than the wound is wide. Therefore, retractors having pre-fixed blades must compromise the blade contour to fit in the wound by having less divergent and less angulated blades. Such a configuration significantly diminishing the ability to function as a deep wound retractor. Thus, spinal retractors with attachable blades have been devised.

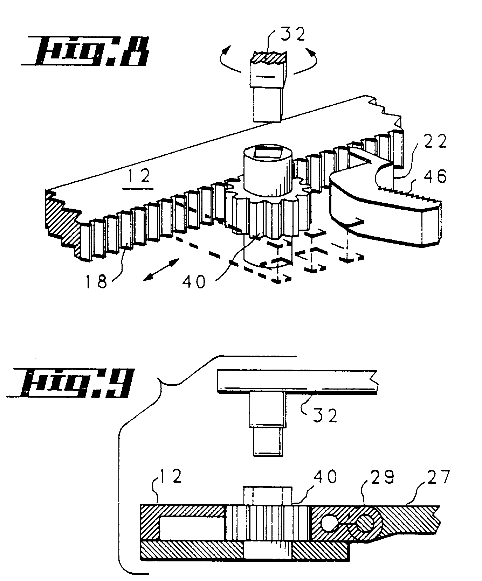

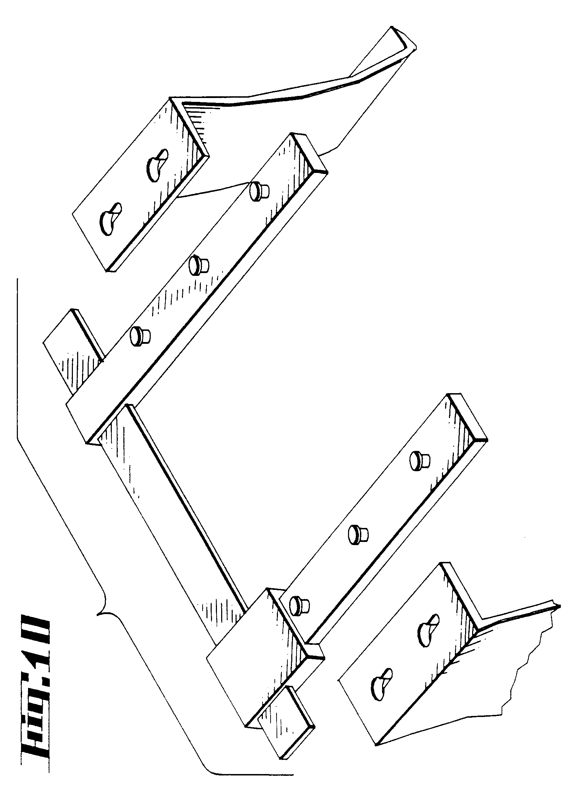

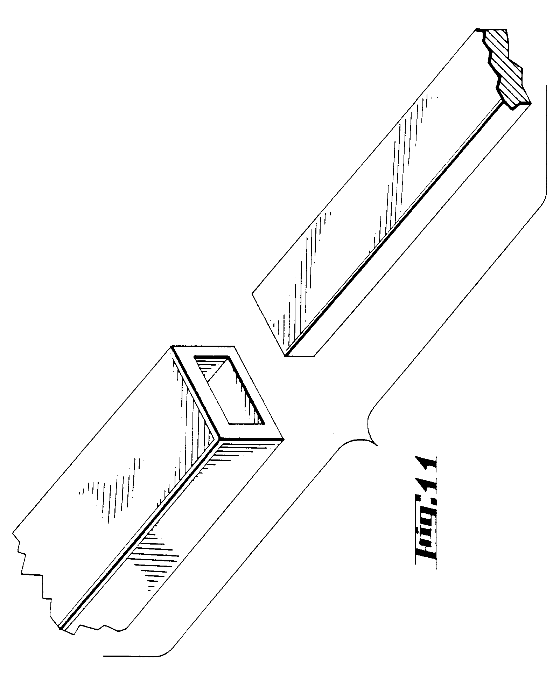

At present, there are essentially two types of large spinal retractors with attachable and removable blades. Such retractors and blades are shown in Figures 10 and 11.

The first type (shown in Figure 11) consists of a blade with a rectangular shaped opening at the top into which the retractor arms can be slid to hold the blade. While this does allow the blade to be inserted into the wound first and the retractor arms then attached, there can be great difficulty in attaching the blades to the retractor arms as both blades must be engaged simultaneously which requires that the blades and the arms of the retractor be opened to exactly the same width. Further, the blades must be parallel to each other and to the retractor and at the same time must protrude from the wound to exactly the same height. Further, the blades must be absolutely perpendicular to the spine, and not be rotated. Achieving all of these conditions at the same time can be quite difficult and time consuming.

There is also a retractor set which uses a rectangular open box-like attachment method, where the blades are strips of metal of similar widths and different lengths such that a series of strips are attached to each arm until a whole blade is essentially constructed, contoured to the particular patient. However, since the blades are attached to the retractor prior to insertion, the blades cannot be optically divergent thereby defeating at least one of the two major advantages sought to be obtained from the use of the attachable blades.

The second type of large spinal retractor currently in use (Figure 10) utilizes a set of attachment posts protruding from the retractor arms which then engage a series of corresponding holes on the top surface of the blades. However, when these blades are placed within the wound first and then an attempt is made to attach the retractor, there is considerable difficultly in aligning the blades and achieving the necessary three dimensional parallelism so as to attach the blades to the retractor. Furthermore, the exposed pegs and locking mechanism pose a threat to the surgeon as it is possible to rupture a glove on the exposed mechanism.

In US-A-4 747 394 there is described a surgical retractor according to the preamble of claim 1 and a blade for use with a surgical retractor according to the preamble of claim 12. More particularly, US-A-4 747 394 discloses a spinal retractor of the first type described above wherein the blades have a rectangular shaped opening at the top into which the retractor arms must be simultaneously slid to hold the blades which can be a very cumbersome and time consuming operation.

US-A-3766910 discloses interchangeable retractor blades sliding in a mating groove in the ends of the retractor arms.

US-A-2053868 discloses a blade forming a three sided groove to fit snugly and slidingly over the bar of a self-retaining retractor.

It is a purpose of the present invention to provide for a spinal retractor and blade set that is easier and faster to use.

It is another purpose of the present invention to provide an improved spinal retractor and blade set that is more efficient, both in executing its purpose and in its method of attachment of the blades to the retractor.

It is another purpose of the present invention to provide for an improved spinal retractor and blade set that is modular in design so as to allow pairs of blades to be assembled in various combinations to enhance the overall variability.

It is still another purpose of the present invention to provide for an improved spinal retractor and blade set that is stronger than any presently existent spinal retractor.

It is yet another purpose of the present invention to provide for an improved spinal retractor and blade set that is of a low profile and without surface projections so as to minimize interference with, and risk to, the surgeon.

It is another purpose of the present invention to provide for an improved spinal retractor and blade set in which the blades divergence is beyond previously existing retractors.

It is another purpose of the present invention to provide for an improved spinal retractor and blade set that is capable to opening a wound more widely than any previously existent spinal retractor.

To achieve this, the surgical retractor of the invention comprises the features claimed in the characterizing part of claim 1 and the blade for use with a surgical retractor is characterized by the features of the characterizing part of claim 12.

According to the invention, the surgical retractor forms a means for aligning the separable blades, the aligning means comprising a narrowed portion in the shape of a depressed cut out segment at least substantially the thickness of a top, slot-defining portion of the blades along substantially the length of the arms along the outer portion of the arms.

More particularly, the present invention is a spinal retractor consisting of a large gear operated frame and a series of detachable, modular, and complementary blades especially designed to facilitate attachment of the blades to the retractor without compromise to the angulation of the blades and consistent with optimal function. The retractor frame has a solid spine which is ratcheted, and one fixed arm and one movable arm attached perpendicularly to the spine. The movable arm is moved by a gear driven mechanism, and secured with a spring loaded lock which engages the ratchet of the spine. The retractor is opened and closed with a handle that is removable so that it will protrude during the surgery.

Both arms are hinged proximate the junction to the frame to allow the retractor frame and its arms to contour to the shape of the patient, assuring a low profile when in use.

The blades are widely divergent from each other distally when placed in a wound and have teeth splaying outward into the wound beyond the maximum divergence point of the blades. The teeth are substantially at right angle to a substantially planar member of the blades. The arms have a depression or cutout portion along one side of substantially their entire length. The blades have a substantially U-shaped configuration along their upper portion for engaging the corresponding depression or cutout on the arms. The separation of the arms results in the automatic, three dimensional alignment of the blades so as to firmly engage the blades on the arms.

### BRIEF DESCRIPTION OF THE DRAWING

Fig. 1 is a perspective view of the retractor.

Figs. 2a, 2b and 2c are perspective views of various blade configurations used with the retractor of Figure 1.

Fig. 3 is a perspective view of the retractor and blades in relation to the spine demonstrating the use of the hand crank to open the retractor.

Fig.4 is a cross sectional view of the lumbar spine demonstrating the cross sectional relationship between the retractor blades and the retractor arms.

Fig 5 is a top view of the retractor and blades situated in the thoracic spine.

Fig 6 is a partial sectional view of the retractor ratchet mechanism and lock mechanism.

Fig. 7 is a partial sectional view of the "fixed" arm.

Fig. 8 is a partial sectional view of the ratchet and lock mechanism.

Fig. 9 is a side sectional view of the hand crank and its relationship to the gear.

Fig. 10 is a perspective view of one version of the prior art.

Fig. 11 is a perspective view of a second version of the prior art.

### DETAILED DESCRIPTION OF THE DRAWINGS

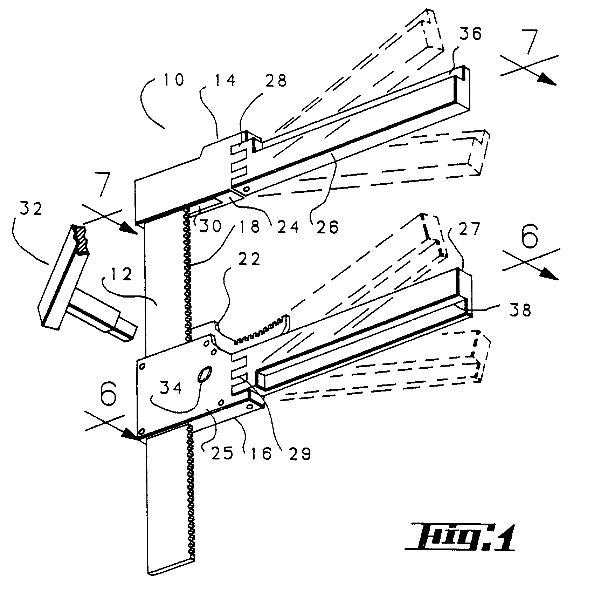

Referring to figure 1 the retractor frame 10 is shown in perspective view. The retractor frame 10 comprises a spine member 12 having a rectangular cross section. A first fixed arm 14 is affixed to the upper portion of the spine member 12 and a second movably arm 16 is movably attached to the spine member 12. The two arms 14 and 16 have a substantially rectangular cross section and parallel to one another and in the same plane.

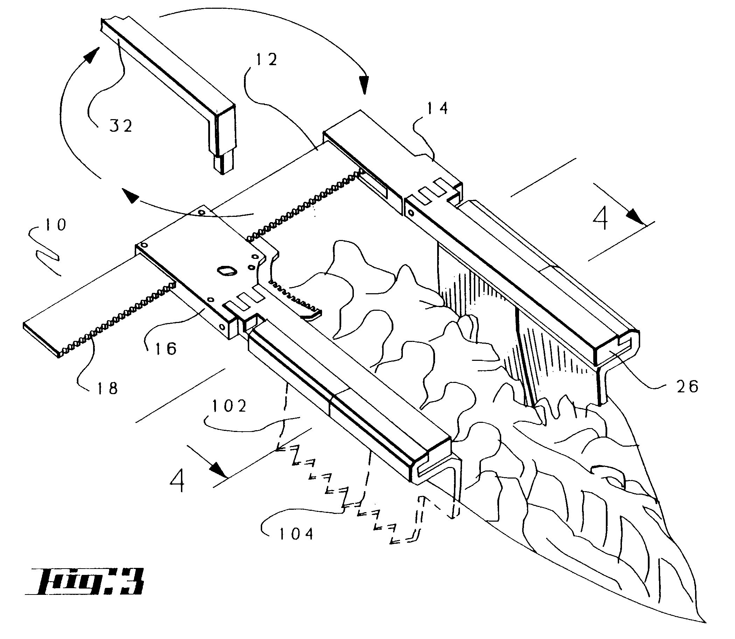

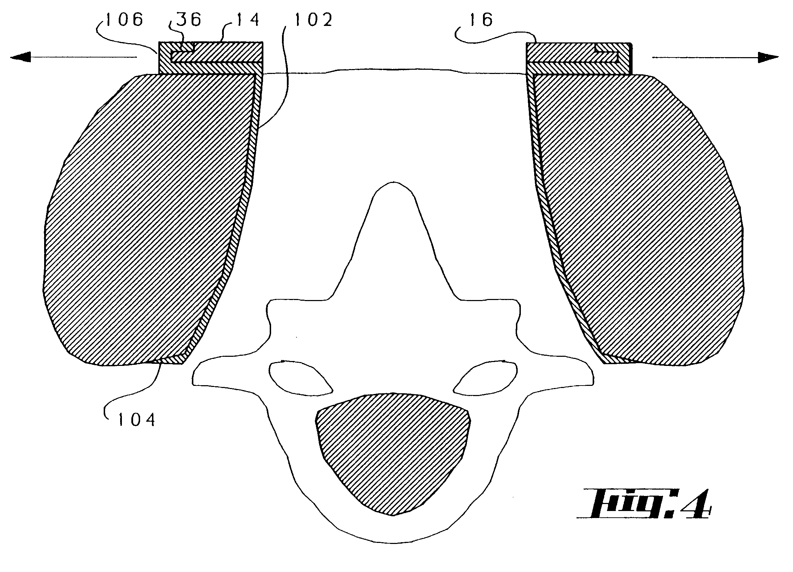

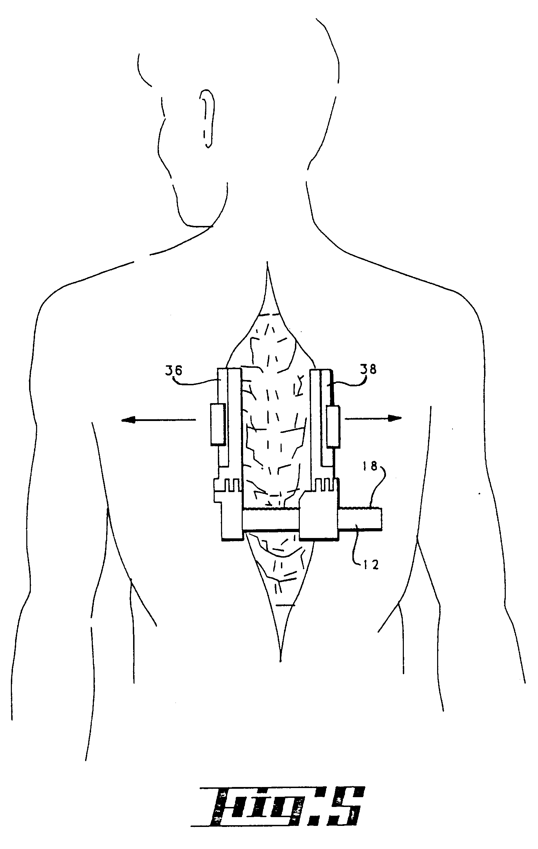

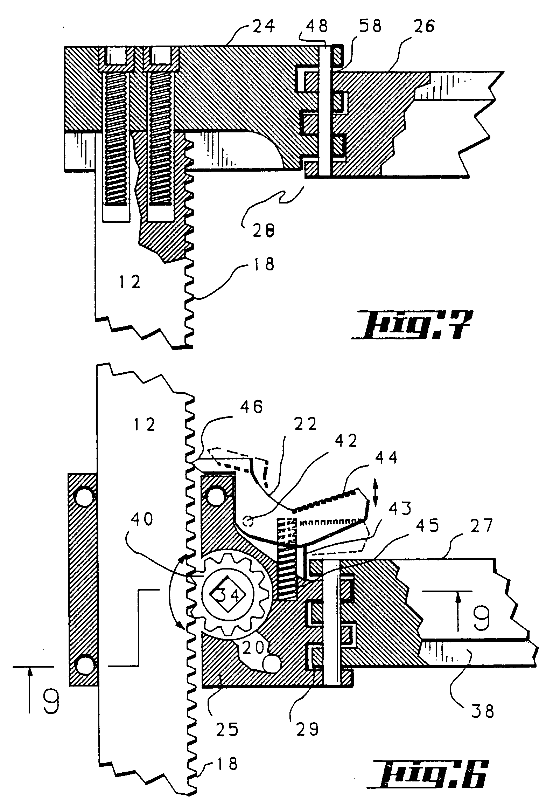

The spine member 12 has a ratchet 18 along its inside surface, which engages a complementary ratchet mechanism on the movable arm 16. The ratchet mechanism 20 (shown in figures 6, 8 and 9) on the movable am is lockable by lock 22 against movement.

The two arms 14 and 16 consists of a first rigid portion and a second hinged portion 26 and 27. The hinged portion 26 and 27 are connected to the first rigid portion 24 and 25 by hinges 28 and 29.

The hinged portions 26 and 27 have an elongated cutout or groove portion 36 and 38 along opposing sides of the hinged portions of the arms. The cutout or depressions extend substantially along the entire length of the edge of the hinged portion 26 and 27 of arms 14 and 16.

A hand crank 32 engages the drive gear opening 34 in the ratchet mechanism 20 for turning the gear mechanism to cause the movable arm 16 to move up and down the spine member 12.

Referring to Figures 6, 8 and 9, the ratchet mechanism 20 is shown in detail. The ratchet mechanism 20 consists of drive gear 40 having a drive gear opening 34 for engagement with the crank 32. The drive gear 40 is complementary to the ratchet 18 along the spine member 12.

The lock 22 is pivotable about pivot pin 42 and biased in a position wherein the lock engages the ratchet 18 by spring 43 enclosed within recess 45 in the rigid portion 25 of arm 16. A thumb depressing portion 44 is at one end of the lock and the other end of the lock 22 consists of a ratchet engaging tip 46 complementing the ratchet 18 of the spine member 12, and biased at an angle so that the lock prevents closing of the arms when engaged, but not the opening of the arms.

Referring to Figure 7, the detail of the hinge 28 is shown. The hinge in both the fixed arm 14 and the movable arm 16 consists of a central pin 48 passing through larger aligned openings 58. The pin 48 may be held in place by screw threads or other conventional means.

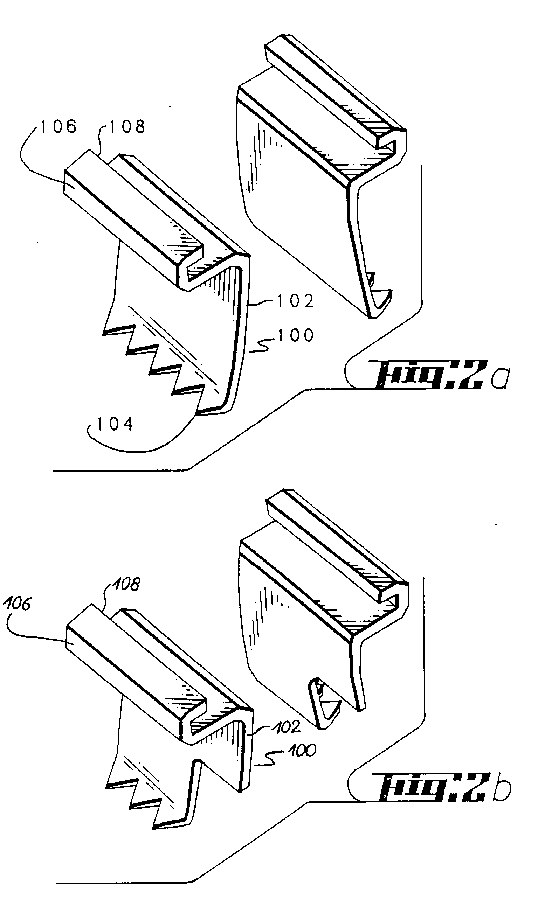

Referring to Figure 2(a) -2(c) alternative forms of blades are shown for use with the retractor. The blades 100 are used in pairs. One blade is used in association with the fixed arm 14 of the retractor frame 10 and the second blade is used in association with the movable arm 16 of the retractor 10.

The blade 100 has a substantially central planar portion 102, substantially perpendicular teeth 104 on the lower end and a U-shaped engaging portion 106 at its upper end. The depth of the U-shaped engaging portion 106 of the blade 100 is approximately 1.9 cm (3/4 inches), slightly smaller than the width of the cutout 36 and 38 of the retractor arms 14 and 16. In the preferred embodiment the cutout has a depth of approximately 1.3 cm (1/2 inch). The teeth 104 are substantially perpendicular to the planar surface 102 and are approximately 1.3 cm (1/2 inch) long.

The blades are also made of heavy stainless steel, so as to resist bending by the muscles. The blades themselves are approximately 0.6 cm (1/4 inch) thick and are approximately 10.2 cm (4 inches) wide.

The spine member 12 is approximately 0.30 m (one foot) long and the maximum extension of the opening between the arms in use is approximately 15.2 cm (6 inches). The length of the arms are approximately 15.2 cm (6 inches) long.

The recess or cutout portion 36 and 38 permits the arms 14 and 16 when the blades 100 are attached to the arms 36 and 38. Otherwise, the upper leg 108 of the blades 100 would extend beyond the top surface of the arms 36 and 38.

The width of the planar portion at the lower end may vary depending on the particular application of which the blades are being used, such as is shown in Figures 2b and 2c.

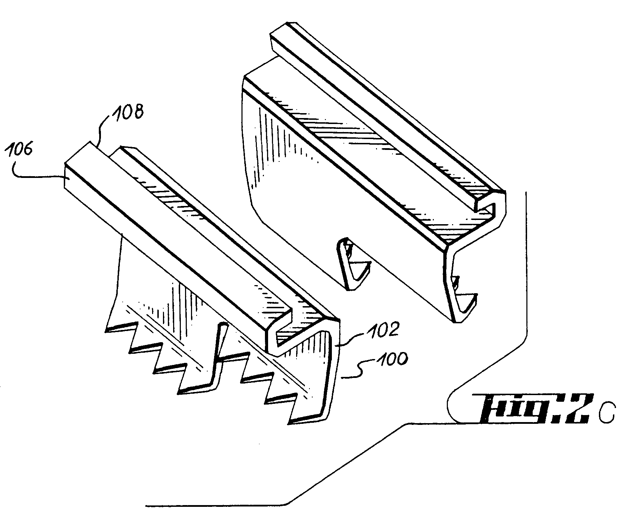

Referring to figures 3-5 the apparatus is shown in use on the patient. In Figure 3 the retractor is shown in the spine, with the hand crank 32 about to be engaged. The teeth of the blades are illustrated as holding the muscles out of the way. In Figure 4 the blades are shown in side sectional view, showing in detail the engagement of the arms with the blades. In Figure 5, a top view of the retractor frame and blades during an operation.

### METHOD OF USE

Blades of the appropriate depth as determined by the size of the patient are inserted on either side of the spine against and beneath the spinal musculature. The blades are inserted in pairs on opposite sides of the wound. The retractor frame is then placed between the blades in a close position. The retractor is opened and when any part of the retractor arm rides over the inner portion of the U-shaped blade engagement area, the retractor arm will then de-rotate, align, and engage the blades as the retractor is opened. To remove the retractor, the crank is again inserted. The lock mechanism is released and the retractor allowed to close until the blades are disengaged. The retractor may then be removed, followed by the blades.

In the preferred embodiment, the retractor is made of heavy metal, such as stainless steel giving the retractor substantial weight to resist the torques that are applied by the muscle of the body.

While the present invention has been described in reference to the preferred embodiment of the invention, it is recognized that other embodiments of the present invention may be made without departing from the present inventive concept.

## Claims

1. A surgical retractor (10) in combination with separable blades (100) Comprising a substantially planar member,
the retractor (10) Comprising: a spine member (12), said spine member (12) having a pair of arms (14, 16) movably attached substantially perpendicular to said spine member (12) and parallel to one another, both of said arms (14, 16) having means (26, 27) for removably holding separable blades (100),
characterized in that said blade holding means (26, 27) forms a means (36, 38) for aligning said separable blades (100), said aligning means (36, 38) comprising a narrowed portion in the shape of a depressed cut out segment (36, 38) at least substantially the thickness of a top, slot-defining portion (106, 108) of said blades (100) along substantially the length of said arms (14, 16) along the outer portion of said arms (14, 16).

2. The combination of claim 1, characterized in that one (14) of said arms (14, 16) is fixed in relationship to said spine member (12) and the other (16) of said arms (14, 16) is movable in relationship to said spine member (12), and both of said arms (14, 16) have a hinged portion (28, 29) permitting movement of a portion (26, 27) of said arms (14, 16) of the plane of the arms (14, 16) and spine member (12).

3. The combination of claim 2, characterized in that said movable arm (16) is movable by a gear mechanism (40).

4. The combination of claim 1, characterized in that said arms (14, 16) have a substantially rectangular cross section and said narrowed portion (36, 38) is along the side of each arm (14, 16) away from the other arm (14, 16).

5. The combination of claim 1, characterized in including at least one pair of blades (100), each of said blades (100) having alignment means (106, 108) for engaging said aligning means (36, 38) on said arms (14, 16), said blades (100) engaging the narrowed portion of said arms (14, 16).

6. The combination of claim 5, characterized in that at least one of said blades (100) comprises a substantially planar member (102), said planar member (102) having teeth (104) at one end and said aligning means (106, 108) being U-shaped.

7. The combination of claim 6, characterized in that said U-shaped aligning means (106, 108) comprises an upper portion (108), a side portion (106) and a bottom portion, defining an opening on one side of said U-shaped aligning means (106, 108), said opening of said U-shaped aligning means (106, 108) faces of said arms (14, 16) and said U-shaped aligning means (106, 108) extends substantially along the entire length of at least one of said blades (100).

8. The combination of claim 7, characterized in that said U-shaped aligning means (106, 108) has an upper leg (108) and a lower leg, the lower leg of said U-shaped aligning means being longer than the upper leg (108), and said legs of said U-shaped aligning means (106, 108) are substantially perpendicular to said planar member (102).

9. The combination of claim 8, characterized in that said blades (100) are maintained on said arms (14, 16) in a perpendicular orientation in relationship to the arms (14, 16).

10. The combination of claim 8, characterized in that the length of the upper leg (108) is less than the depth of the narrowed portion (36, 38) as measured normal to one of said arms (14, 16).

11. The combination of claim 1, characterized in that said arms (14, 16) have a hinged portion (28, 29).

12. A surgical retractor (10) in combination with a blade (100), said surgical retractor (10) having arms (14, 16) for removably holding the blades (100), and said blade (100) comprising a substantially planar member (102) having teeth (104) at one end,
characterized in said blade (100) further comprises a U-shaped engaging member (106, 108) at the other end of said planar member (102), said U-shaped engaging member (106, 108) forming a top, slot-defining portion (106, 108) of the blade (100) for engaging one of the arms (14, 16).

13. The combination of claim 12, characterized in that said U-shaped engaging member (106, 108) has a width slightly larger than the width of the arms (14, 16) of the retractor (10).

14. The combination of claim 12, characterized in that the legs (108) of said U-shaped engaging member (106, 108) are perpendicular to said planar member (102).

15. The combination of claim 12, characterized in that said U-shaped engaging member (106, 108) maintains said blade (100) in a perpendicular orientation in relationship to the arms (14, 16) of the retractor (10).

## Patentansprüche

1. Chirurgische Rückhaltevorrichtung (10) in Kombination mit abtrennbaren Blättern (100), die einen im wesentlichen ebenen Teil umfassen,
wobei die Rückhaltevorrichtung (10) umfasst: ein Rückenteil (12), wobei das Rückenteil (12) ein Paar von Armen (14, 16) hat, die beweglich im wesentlichen senkrecht zum Rückenteil (12) und parallel zueinander befestigt sind, wobei beide der Arme (14, 16) eine Einrichtung (26, 27) für die wieder entfernbare Befestigung von abtrennbaren Blättern (100) haben,
dadurch gekennzeichnet, dass die Befestigungseinrichtung (26, 27) für die Blätter eine Einrichtung (36, 38) bildet, um die abtrennbaren Blätter (100) auszurichten, wobei die ausrichtende Einrichtung (36, 38) entlang im wesentlichen der Länge der Arme (14, 16) entlang dem äusseren Teil der Arme (14, 16) eine sich verengende Partie in der Form eines vertieften ausgeschnittenen Segmentes (36, 38) mit mindestens im wesentlichen der Dicke einer oberen, einen Schlitz definierenden Partie (106, 108) der Blätter (100) umfasst.

2. Kombination nach Anspruch 1, dadurch gekennzeichnet, dass einer (14) der Arme (14, 16) bezüglich dem Rückenteil (12) fixiert ist, und der andere (16) der Arme (14, 16) bezüglich dem Rückenteil (12) beweglich ist, und beide der Arme (14, 16) eine Scharnierpartie (28, 29) haben, welche die Bewegung eines Teils (26, 27) der Arme (14, 16) aus der Ebene aus den Armen (14, 16) und dem Rückenteil (12) ermöglicht.

3. Kombination nach Anspruch 2, dadurch gekennzeichnet, dass der bewegliche Arm (16) über eine Zahnradvorrichtung (40) bewegt werden kann.

4. Kombination nach Anspruch 1, dadurch gekennzeichnet, dass die Arme (14, 16) einen im wesentlichen rechteckigen Querschnitt haben und die sich verengende Partie (36, 38) sich entlang der vom anderen Arm weg gerichteten Seite von jedem Arm (14, 16) befindet.

5. Kombination nach Anspruch 1, dadurch gekennzeichnet, dass sie mindestens ein Paar von Blättern (100) umfasst, wobei jedes der Blätter (100) eine Ausrichtungseinrichtung (106, 108) hat, um in die ausrichtende Einrichtung (36, 38) auf den Armen (14, 16) einzugreifen, wobei die Blätter (100) in die sich verengende Partie der Arme (14, 16) eingreifen.

6. Kombination nach Anspruch 5, dadurch gekennzeichnet, dass mindestens eines der Blätter (100) eine im wesentlichen ebene Partie (102) umfasst, wobei die ebene Partie (102) an einem Ende Zähne (104) hat und die Ausrichtungseinrichtung (106, 108) U-förmig ist.

7. Kombination nach Anspruch 6, dadurch gekennzeichnet, dass die U-förmige Ausrichtungseinrichtung (106, 108) einen oberen Teil (108), einen Seitenteil (106) und einen Bodenteil umfasst, die eine Öffnung auf der einen Seite der U-förmigen Ausrichtungseinrichtung (106, 108) definieren, wobei die Öffnung der U-förmigen Ausrichtungseinrichtung (106, 108) gegen die Arme (14, 16) zeigt und sich die U-förmige Ausrichtungseinrichtung (106, 108) im wesentlichen entlang der gesamten Länge von mindestens einem der Blätter (100) erstreckt.

8. Kombination nach Anspruch 7, dadurch gekennzeichnet, dass die U-förmige Ausrichtungseinrichtung (106, 108) ein oberes Bein (108) und ein unteres Bein hat, wobei das untere Bein der U-förmigen Ausrichtungseinrichtung länger ist als das obere Bein (108) und die Beine der U-förmigen Ausrichtungseinrichtung (106, 108) im wesentlichen senkrecht zur ebenen Partie (102) sind.

9. Kombination nach Anspruch 8, dadurch gekennzeichnet, dass die Blätter (100) auf den Armen (14, 16) in einer senkrechten Ausrichtung bezüglich der Arme (14, 16) festgehalten werden.

10. Kombination nach Anspruch 9, dadurch gekennzeichnet, dass die Länge des oberen Beines (108) kleiner ist als die Tiefe der sich verengenden Partie (36, 38), wie sie senkrecht zu einem der Arme (14, 16) gemessen wird.

11. Kombination nach Anspruch 1, dadurch gekennzeichnet, dass die Arme (14, 16) eine Scharnierpartie (28, 29) haben.

12. Chirurgische Rückhaltevorrichtung (10) in Kombination mit einem Blatt (100), wobei die chirurgische Rückhaltevorrichtung (10) Arme (14, 16) für die wieder entfernbare Befestigung der Blätter (100) hat und das Blatt (100) eine im wesentlichen ebene Partie (102) umfasst, welche Zähne (104) an einem Ende hat,
dadurch gekennzeichnet, dass das Blatt (100) weiter eine U-förmiges Befestigungsteil (106, 108) am anderen Ende der ebenen Partie (102) umfasst, wobei das U-förmiges Befestigungsteil (106, 108) eine obere, einen Schlitz definierenden Partie (106, 108) des Blattes (100) für die Befestigung an einem der Arme (14, 16) bildet.

13. Kombination nach Anspruch 12, dadurch gekennzeichnet, dass das U-förmige Befestigungsteil (106, 108) eine Breite hat, die ein wenig grösser ist, als die Breite der Arme (14, 16) der Rückhaltevorrichtung (10).

14. Kombination nach Anspruch 12, dadurch gekennzeichnet, dass die Beine (108) des U-förmigen Befestigungsteils (106, 108) senkrecht zur ebenen Partie (102) stehen.

15. Kombination nach Anspruch 12, dadurch gekennzeichnet, dass das U-förmige Befestigungsteil (106, 108) das Blatt (100) in einer senkrechten Ausrichtung bezüglich der Arme (14, 16) der Rückhaltevorrichtung (10) festhält.

## Revendications

1. Un rétracteur chirurgical (10) en combinaison avec des lames séparables (100) comprenant un élément essentiellement plat,
le rétracteur (10) comprenant: un élément dorsal (12), ledit élément dorsal (12) ayant une paire de bras (14,16) attachés de façon mobile essentiellement perpendiculaires audit élément dorsal (12) et parallèles l'un par rapport à l'autre, les deux bras en question (14,16) ayant un moyen (26,27) pour maintenir de façon amovible les lames séparables (100),
caractérisé en ce que ledit moyen maintenant les lames (26,27) forme un moyen (36,38) pour aligner lesdites lames séparables (100), ledit moyen d'alignement (36,38) comprenant une portion rétrécie sous la forme d'un segment découpé abaissé (36,38) au moins essentiellement de l'épaisseur d'une portion supérieure, définissant une fente (106,108) desdites lames (100) le long essentiellement de la longueur desdits bras (14,16) le long de la portion externe desdits bras (14,16).

2. La combinaison de la revendication 1, caractérisée en ce que un (14) desdits bras (14,16) est fixé par rapport audit élément dorsal (12) et l'autre (16) desdits bras (14,16) est mobile par rapport audit élément dorsal (12) et les deux bras en question (14,16) ont une portion de charnière (28,29) permettant un mouvement d'une portion (26,27) desdits bras (14,16) par rapport au plan des bras (14,16) et de l'élément dorsal (12).

3. La combinaison de la revendication 2, caractérisée en ce que ledit bras mobile (16) est mobile par un mécanisme d'engrenage (40).

4. La combinaison de la revendication 1, caractérisée en ce que lesdits bras (14,16) ont une section transversale essentiellement rectangulaire et ladite portion rétrécie (36,38) est le long du côté de chaque bras (14,16) à l'écart de l'autre bras (14,16).

5. La combinaison de la revendication 1, caractérisée en ce qu'elle comprend au moins une paire de lames (100) chacune desdites lames (100) ayant un moyen d'alignement (106,108) pour engager ledit moyen d'alignement (36,38) sur lesdits bras (14,16), lesdites lames (100) engageant la portion rétrécie desdits bras (14,16).

6. La combinaison de la revendication 5, caractérisée en ce que au moins une desdites lames (100) comprend un élément essentiellement plat (102), ledit élément plat (102) ayant des dents (104) au niveau d'une extrémité et ledit moyen d'alignement (106,108) étant façonné en U.

7. La combinaison de la revendication 6, caractérisée en ce que ledit moyen d'alignement façonné en U (106,108) comprend une portion supérieure (108), une portion latérale (106) et une portion de base, définissant une ouverture sur un côté dudit moyen d'alignement façonné en U (106,108), ladite ouverture dudit moyen d'alignement façonné en U (106,108) fait face auxdits bras (14,16) et ledit moyen d'alignement façonné en U (106,108) s'étend essentiellement sur toute la longueur d'au moins une desdites lames (100).

8. La combinaison de la revendication 7, caractérisée en ce que ledit moyen d'alignement façonné en U (106,108) a une jambe supérieure (108) et une jambe inférieure, la jambe inférieure dudit moyen d'alignement façonné en U étant plus longue que la jambe supérieure (108) et lesdites jambes dudit moyen d'alignement façonné en U (106,108) sont essentiellement perpendiculaires audit élément plat (102).

9. La combinaison de la revendication 8, caractérisée en ce que lesdites lames (100) sont maintenues sur lesdits bras (14,16) dans une orientation perpendiculaire par rapport aux bras (14,16).

10. La combinaison de la revendication 8, caractérisée en ce que la longueur de la jambe supérieure (108) est inférieure à la profondeur de la portion rétrécie (36,38) telle que mesurée perpendiculairement à un desdits bras (14,16).

11. La combinaison de la revendication 1, caractérisée en ce que lesdits bras (14,16) ont une portion de charnière (28,29).

12. Un rétracteur chirurgical (10) en combinaison avec une lame (100),ledit rétracteur chirurgical (10) comprenant des bras (14,16) pour maintenir de façon amovible les lames (100), et ladite lame (100) comprenant un élément essentiellement plat (102) ayant des dents (104) à une extrémité,
caractérisé en ce que la lame en question (100) comprend en outre un élément d'engagement en forme de U (106,108) à l'autre extrémité dudit élément plat (102), ledit élément d'engagement en forme de U (106,108) formant une portion supérieure de la lame (100) définissant une fente (106,108) pour engager un des bras (14,16).

13. La combinaison de la revendication 12, caractérisée en ce que ledit élément d'engagement en forme de U (106,108) à une largeur légèrement plus grande que la largeur des bras (14,16) du rétracteur (10).

14. La combinaison de la revendication 12, caractérisée en ce que les jambes (108) dudit élément d'engagement en forme de U (106,108) sont perpendiculaires audit élément plat (102).

15. La combinaison de la revendication 12, caractérisée en ce que ledit élément d'engagement en forme de U (106,108) maintient ladite lame (100) dans une orientation perpendiculaire par rapport aux bras (14,16) du rétracteur (10).
